# DEMANDE DE BREVET EUROPEEN

(11) **EP 1 493 740 A1**
(43) Date de publication de la demande: **05.01.2005**
(21) Numéro de dépôt: 03291642.1
(22) Date de dépôt: 03.07.2003
(51) Int. Cl.: C07D 333/38, C07D 409/10, A61K 31/381, A61P 11/00, A61P 35/00, A61P 19/00, A61P 9/10

(54) **Dérivés de 5-fluoro-thiophene, leur procédé de preparation, les compositions pharmaceutiques les contenant et leur utilisation comme inhibiteurs de métalloprotéinases**

(71) Demandeur: Warner-Lambert Company LLC, Morris Plains, New Jersey 07950 (US)
(72) Inventeur: Compere, Delphine, c/o Pfizer Pgrd - Patent Dept., 94265 Fresnes Cedex (FR); Dublanchet, Anne-C., c/o Pfizer PGRD-Patent Dept., 94265 Fresnes Cedex (FR); Courte, Karine, c/o Pfizer Pgrd - Patent Dept., 94265 Fresnes Cedex (FR); Blais, Stéphane, c/o Pfizer Pgrd - Patent Dept., 94265 Fresnes Cedex (FR)
(74) Mandataire: Dekker, Henrike

(57) **Abrégé**

Composés de formule (I) : 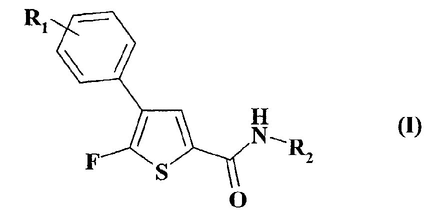 dans laquelle :
- R₁ représente un groupement choisi parmi halogéno(C₁-C₆)alkoxy, (C₁-C₆)alkyle, (C₁-C₆)alkoxy, (C₁-C₆)alkylthio, phényle, cyclohexyle, et un hétérocycle, chacun de ces groupements étant éventuellement substitués par un à deux groupements tels que définis dans la description,
- R₂ représente un groupement -U-R₄ dans lequel :
   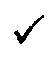 U représente une chaîne (C₁-C₄)alkylène linéaire éventuellement substituée par un groupement chosi parmi carboxy, carboxy(C₁-C₆)alkyl-, (C₁-C₆)alkyloxycarbonyl et (C₁-C₆)alkyloxycarbonyl(C₁-C₆)alkyl-,
      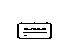 R₄ représente un groupement phényle, cyclohexyle, ou morpholin-4-yl chacun de ces groupements étant éventuellement substitué par un ou deux groupements tels que définis dans la description,
leurs isomères optiques ou leurs sels d'addition à un acide ou à une base pharmaceutiquement acceptable,
et les compositions pharmaceutiques les contenant utiles pour le traitement des pathologies liées à une inhibition des métalloprotéinases, et plus spécifiquement à une inhibition de la métalloprotéinase-12.

## Description

La présente invention concerne des dérivés de fluoro-thiophène, leur procédé de préparation et les compositions pharmaceutiques qui les contiennent.

Les composés de la présente invention sont particulièrement intéressants d'un point de vue pharmacologique pour leur interaction spécifique avec les métalloprotéinases et plus spécifiquement avec la métalloélastase macrophagique (MMP-12), trouvant leur application dans la prévention et le traitement des pathologies respiratoires telles que les broncho-pneumopathies chroniques obstructives (BPCO), l'emphysème, les bronchites chroniques, les inflammations pulmonaires chroniques, l'asthme, la fibrose cystique, la détresse respiratoire aiguë (ARDS), les allergies respiratoires dont la rhinite allergique, ainsi que les maladies liées à la production de TNFα incluant les maladies pulmonaires fibrotiques sévères, la sarcoïdose pulmonaire, et la silicose. Les composés de la présente invention montrent aussi, à un niveau moindre, une activité inhibitrice de la métalloprotéinase-13 (MMP-13) les rendant potentiellement utiles pour le traitement des pathologies impliquant cette enzyme, telles que le cancer, l'ostéoporose, l'ostéoarthrite, l'arthrite, l'arthrite rhumatoïde, l'athéorosclérose, la sclérose en plaques, l'insuffisance cardiaque.

Les métalloprotéinases (MMPs) constituent une large famille de proteinases dégradant la matrice extracellulaire et sont sécrétées notamment par les cellules mésenchymales, les macrophages et les leucocytes polynucléaires. Les métalloprotéinases sont classées en plusieurs sous-familles selon leur structure primaire et leur spécificité. Ces familles incluent notamment les collagénases (MMP-1, MMP-8 et MMP-13), les stromélysines (MMP-3 et MMP-10), les gélatinases (MMP-2 et MMP-9), la matrilysine (MMP-7), la métalloélastase macrophagique (MMP-12) ainsi que les MMPs de type membranaire (MMP-14, MMP-15, MMP-16 et MMP-17).

Les MMPs sont des zinc-métalloprotéinases ayant la capacité de dégrader la quasi-totalité des composants de la matrice extracellulaire, c'est-à-dire l'interstitium et les membranes basales. Une synthèse accrue de ces enzymes est retrouvée dans de nombreuses maladies destructrices (arthrite inflammatoire, athérosclérose, invasion tumorale, angiogénèse). Les MMPs (en particulier celles ayant une puissante activité élastolytique) sont impliquées dans la physiopathologie de l'asthme et des broncho-pneumopathies chroniques obstructives dont l'emphysème pulmonaire lié au tabac (BPCO).

L'élastase macrophagique humaine (HME ou MMP-12) présente toutes les caractéristiques des autres MMPs. Elle dégrade de nombreuses macromolécules de la matrice extracellulaire (gélatine, fibronectine et laminine) et surtout l'élastine. La MMP-12 n'est pas synthétisée par les cellules monocytaires circulantes, mais uniquement par les macrophages ou encore les monocytes différenciés *in vitro* en macrophages. La pathologie de l'emphysème est caractérisée par la destruction de l'élastine présente dans les parois des alvéoles pulmonaires. La mise en évidence de l'augmentation du taux de MMP-12 lors de la manifestation de cette pathologie, suggère ainsi un rôle prédominant de cette enzyme dans la survenue et le développement de cette maladie. De même des études ont démontré l'absence de développement d'emphysème chez des souris déficientes en MMP-12, ces souris étant exposées longuement à la fumée de cigarette (*Science* **1997**, 277, 2002-2004). Plus récemment, en utilisant également des souris déficientes en MMP-12, dans un modèle d'asthme, un groupe a suggéré l'implication de la MMP-12 dans le développement de l'asthme chronique (*FASEB*, **2002,** 16, A590). Ces résultats démontrent clairement que des inhibiteurs de l'élastase macrophagique humaine (MMP-12) pourraient être très utiles pour la prévention et le traitement de pathologies respiratoires chroniques telles que la bronchite pulmonaire chronique obstructive (BPCO), l'emphysème, les bronchites chroniques, les inflammations pulmonaires chroniques, mais également les pathologies respiratoires dues à un phénomène d'inflammation telles que l'asthme, la mucoviscidose, la détresse respiratoire aiguë (ARDS), les allergies respiratoires dont la rhinite allergique, ainsi que les maladies liées à la production de TNFα incluant les maladies pulmonaires fibrotiques sévères, la sarcoïdose pulmonaire, et la silicose.

Toutes les métalloprotéinases présentent un domaine catalytique constitué de 162 à 173 acides aminés contenant le site actif de l'enzyme. Un ion Zn²⁺ est présent dans le site actif auquel il est fixé par l'intermédiaire de résidus histidines. Ce site constitue l'un des points d'ancrage privilégié des inhibiteurs synthétiques des MMPs car il permet notamment de créer un centre de chélation stable, puissant et aisément accessible pour les petites molécules. Ainsi tous les inhibiteurs puissants décrits dans la littérature possèdent une fonction chimique telle qu'un acide hydroxamique permettant une chélation entre l'atome de zinc du site catalytique de la métalloprotéinase et ledit inhibiteur. Cette chélation assure un blocage du site actif et entraîne l'inhibition de ladite enzyme.

L'un des problèmes majeurs de ce type d'inhibition est l'absence de sélectivité ou le faible taux de sélectivité, toutes les MMPs possédant en effet un ion zinc au sein de leur site actif. Le second problème lié à ces inhibiteurs puissants mais généralement faiblement sélectifs est la toxicité liée à la présence d'une fonction chimique telle qu'un acide hydroxamique.

L'un des objets de l'invention est donc de fournir de nouveaux composés possédant des propriétés inhibitrices de la métalloprotéinase de type 12 (MMP-12). Une solution a été trouvée par l'élaboration de nouveaux dérivés fluoro-thiophène, ainsi que par l'utilisation desdits composés dans des compositions pharmaceutiques aptes à être utilisées dans la prévention et le traitement des pathologies liées à une inhibition de la MMP-12.

Plusieurs demandes de brevets ou articles scientifiques décrivent des composés comportant un motif central thiophène. Parmi cette littérature il peut être cité la demande de brevet WO 98/23605 qui décrit des dérivés thién-2-yl-carboxamides substitués en position 4 par un système cyclique et en position 5 par un groupement trifluorométhyle. Ces composés sont revendiqués pour leur activité bactéricide et fongicide. La demande de brevet WO 96/16954 décrit également des composés comportant éventuellement un système 4-aryl-thién-2-yl carboxamide dans lequel la fonction amide peut être substituée par un groupement phényle, utiles pour leur propriété anti-fongique.

Il peut également être cité la demande de brevet JP 63175853 ou l'article *Chem. Commun*. **2001**, 8, 759-760, qui décrivent des composés comportant un groupement thiophène substitué, ces composés constituant des photo-régulateurs de fluorescence ou des révélateurs photographiques.

Aucun de ces documents ne décrit ou ne suggère pour ces composés une activité inhibitrice de la MMP-12 et une utilisation potentielle de ce type de produits dans le traitement de pathologies respiratoires, propriété originale des composés revendiqués par la demanderesse. Plus spécifiquement aucun de ces documents ne décrit des dérivés (5-fluoro-4-phényl)-thièn-2-yl carboxamide en tant qu'inhibiteur de la MMP-12.

Plus particulièrement, la présente invention concerne les composés de formule (I) : 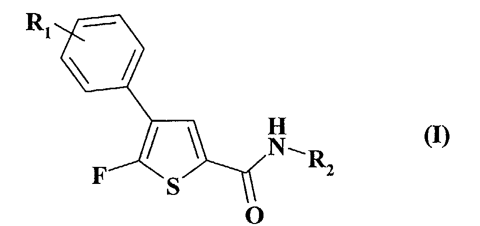 dans laquelle :
- R₁ représente un groupement choisi parmi halogéno(C₁-C₆)alkoxy, (C₁-C₆)alkyle, (C₁-C₆)alkoxy, (C₁-C₆)alkylthio, et -R₃ dans lequel :
   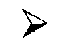 R₃ représente un groupement choisi parmi phényle, cyclohexyle, et un hétérocycle, chacun de ces groupements étant éventuellement substitués par un à deux groupements, identiques ou différents, indépendamment l'un de l'autre choisis parmi halogène, halogéno(C₁-C₆)alkyl-, halogéno(C₁-C₆)alkoxy-, (C₁-C₆)alkyle, cyano, cyano(C₁-C₆)alkyl-, hydroxy, (C₁-C₆)alkoxy, phénoxy, (C₁-C₆)alkyl-SO₂-, (C₁-C₆)alkylcarbonyl, benzoyl, hydroxy(C₁-C₆)alkyl-, et amino éventuellement substitué par un ou deux groupements (C₁-C₆)alkyle identiques ou différents ;
- R₂ représente un groupement -U-R₄ dans lequel :
   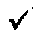 U représente une chaîne (C₁-C₄)alkylène linéaire éventuellement substituée par un groupement choisi parmi carboxy, carboxy(C₁-C₆)alkyl-, (C₁-C₆)alkyloxycarbonyl, (C₁-C₆)alkyloxycarbonyl(C₁-C₆)alkyl-, aminocarbonyl et aminocarbonyl(C₁-C₆)alkyl dans lesquels la partie amino est éventuellement substituée par un ou deux groupements (C₁-C₆)alkyl identiques ou différents,
   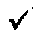 R₄ représente un groupement phényle, cyclohexyle, ou morpholin-4-yl chacun de ces groupements étant éventuellement substitué par un ou deux groupements, identiques ou différents, indépendamment l'un de l'autre choisis parmi halogène, - OR₅, -CO₂R₅, -W-CO₂R₅, dans lesquels :
      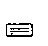 R₅ représente un atome d'hydrogène ou un groupement (C₁-C₆)alkyle,
      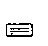 W représente une chaîne (C₁-C₆)alkylène linéaire ou ramifiée,
leurs isomères, ainsi que leurs sels d'addition à un acide ou à une base pharmaceutiquement acceptable ;
étant entendu que dans la définition générale des différents groupements des composés de formule (I):
- par un groupement hétérocycle on comprend un système monocyclique de 5 à 6 chaînons, saturé, insaturé ou aromatique, comprenant de 1 à 2 hétéroatomes, identiques ou différents, choisis parmi oxygène et azote: furyle, pyrrolyle, pyrazolyle, pyridyle, pyrimidyle, pyrazinyle, indolyle, quinolyle, isoquinolyle, imidazolyle, N-pyrrolydinyle, 3,6-dihydro-2H-pyridin-1-yl ,...
- par groupement (C₁-C₆)alkyle on comprend une chaîne carbonée linéaire ou ramifiée comprenant de 1 à 6 atomes de carbone; à titre indicatif il peut être cité les groupements suivants : méthyle, éthyle, propyle, isopropyle, tert-butyle, néopentyle, hexyle,...
- par groupement (C₁-C₆)alkoxy on comprend un groupement alkyle tel que défini précédemment lié au travers d'un atome d'oxygène ; à titre indicatif il peut être cité les groupements suivants : méthoxy, éthoxy, *n*-propyloxy, *tert*-butyloxy,...
- par groupement halogéno(C₁-C₆)alkyle on comprend une chaîne carbonée linéaire ou ramifiée comprenant de 1 à 6 atomes de carbone et substituée par 1 à 6 atomes d'halogène; à titre indicatif il peut être cité les groupements suivants : trifluorométhyle, 2,2,2-trifluoroéthyle, ...
- par groupement halogéno(C₁-C₆)alkoxy on comprend une chaîne carbonée linéaire ou ramifiée comprenant de 1 à 6 atomes de carbone et substituée par 1 à 6 atomes d'halogène, ladite chaîne étant reliée au composé de formule (I) par un atome d'oxygène ; à titre indicatif il peut être cité les groupements suivants : trifluorométhoxy, 2,2,2-trifluoroéthoxy, ...
- par atome d'halogène on comprend un atome choisi parmi brome, chlore, fluor et iode,
- les isomères optiques se réfèrent aux racémates, énantiomères et diastéréoisomères.

Selon une variante particulièrement intéressante de l'invention, le groupement phényle des composés de formule (I) est substitué par un groupement R₁ tel que défini dans la formule (I) situé en position *para.*

Les groupements R₁ préférés selon l'invention sont les groupements choisis parmi trifluorométhoxy, 4-acétylphényle, 4-pyridyle, 3-pyridyle, N-pyrrolydinyle, 1-méthylpyrrol-3-yl, 3,6-dihydro-2H-pyridin-1-yl, 2-hydroxyphényle et 2-hydroxy-4-pyridyle.

D'une façon particulièrement avantageuse R₁ représente un groupement choisi parmi trifluorométhoxy, 4-acétylphényle et 4-pyridyle.

Selon une variante préférée de l'invention, R₂ représente un groupement -U-R₄ dans lequel U représente une chaîne (C₁-C₂)alkylène linéaire et R₄ représente un groupement cyclohexyle substitué en position 4- par un groupement carboxy. Plus spécifiquement les composés préférés de l'invention sont ceux pour lesquels ledit groupement carboxy est de stéréochimie trans.

Selon une autre variante préférée de l'invention, R₂ représente un groupement -U-R₄ dans lequel U représente un groupement méthylène substitué par un groupement carboxyméthyle ou aminocarbonylméthyle, et R₄ représente un groupement phényle. D'une façon particulièrement intéressante l'atome de carbone portant le groupement R₄ prenant la définition phényle est de configuration absolue (*R*).

Les isomères ainsi que les sels d'addition à un acide ou à une base pharmaceutiquement acceptable des variantes et des composés préférés font partie intégrante de l'invention.

L'invention concerne aussi les sels pharmaceutiquement acceptables des composés de formule (I). Une revue des sels pharmaceutiquement acceptables est notamment décrite dans *J*. *Pharm. Sci*., **1977,** 66, 1-19.
Les acides pharmaceutiquement acceptables signifient les acides non toxiques organiques ou minéraux. Parmi les acides pharmaceutiquement acceptables, on peut citer à titre non limitatif les acides chlorhydrique, bromhydrique, sulfurique, phosphonique, nitrique, acétique, trifluoroacétique, lactique, pyruvique, malonique, succinique, glutarique, fumarique, tartrique, maléïque, citrique, ascorbique, oxalique, méthane sulfonique, camphorique, benzoique, toluènesulfonique, etc...

Les bases pharmaceutiquement acceptables signifient les bases non toxiques organiques ou minérales.
Parmi les bases pharmaceutiquement acceptables, on peut citer à titre non limitatif l'hydroxyde de sodium, l'hydroxyde de potassium, l'hydroxyde de lithium, l'hydroxyde de calcium, la triéthylamine, la tertbutylamine, la 2-diéthylaminoéthanol, l'éthanolamine, l'éthylènediamine, la dibenzyléthylènediamine, la piperidine, la pyrrolidine, la morpholine, la piperazine, la benzylamine, l'arginine, la lysine, l'histidine, la glucamine, la glucosamine, les hydroxides d'ammonium quaternaire, etc...

D'une façon générale on comprend par isomères des composés de l'invention, les isomères optiques tels que les énantiomères et les diastéréoisomères. Plus particulièrement, les formes énantiomères pures des composés de l'invention peuvent être séparées à partir des mélanges d'énantiomères qui sont mis à réagir avec un agent libérable de dédoublement des racémiques, ledit agent existant quant à lui sous la forme d'un énantiomère pur, permettant d'obtenir les diastéréoisomères correspondants. Ces diastéréoisomères sont ensuite séparés selon les techniques de séparation bien connues de l'homme de l'art, telles que la cristallisation ou la chromatographie, puis l'agent de dédoublement est éliminé en utilisant les techniques classiques de la chimie organique, pour conduire à l'obtention d'un énantiomère pur. D'une autre façon les formes énantiomères pures des composés de l'invention peuvent être séparées par chromatographie sur colonne chirale.

Les composés de l'invention qui sont présents sous la forme d'un mélange de diastéréoisomères, sont isolés sous forme pure par l'utilisation des techniques classiques de séparation telles que les chromatographies.

Dans certains cas particuliers, le procédé de séparation des composés de l'invention peut conduire à la formation prédominante d'un énantiomère ou d'un diastéréoisomère par rapport à l'autre.

L'invention s'étend également au procédé de préparation des composés de formule (I). Plus particulièrement les composés de formule (I) peuvent être obtenus à partir des composés de formule (II) : 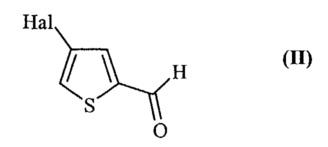 dans laquelle Hal représente un atome d'halogène,
composés de formule (II) qui sont :
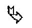 soit mis à réagir, dans des conditions basiques de couplage au palladium, avec un composé de formule (III) : 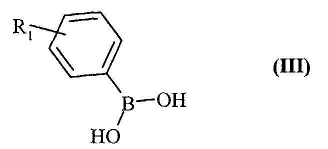 dans laquelle R₁ est tel que défini dans la formule (I),
   pour conduire aux composés de formule (IV) : 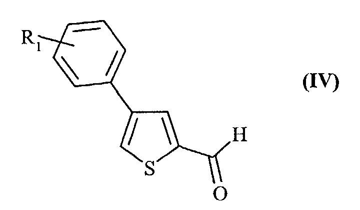 dans laquelle R₁ est tel que défini précédemment,
   composés de formule (IV) qui sont soumis à des conditions d'oxydation en présence, par exemple, de nitrate d'argent en milieu basique et polaire, pour conduire aux composés de formule (V) : 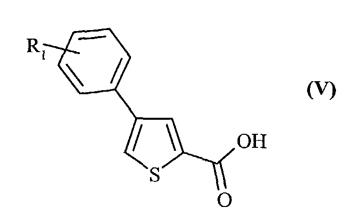 dans laquelle R₁ est tel que défini précédemment,
   composés de formule (V) qui sont traités, dans des conditions de couplage peptidique en présence par exemple d'un agent de couplage et dans un milieu basique avec un composé de formule (VI) :

   H₂N-R₂ (VI)

   dans laquelle R₂ est tel que défini dans la formule (I) :
   pour conduire aux composés de formule (VII) : 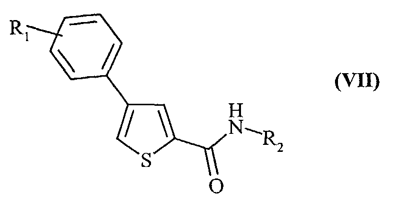 dans laquelle R₁ et R₂ sont tels que définis précédemment,
   composés de formule (VII) qui sont alors traités par du 1-chlorométhyl-4-fluoro-1,4-diazoniabicyclo[2,2,2]octane bis(tetrafluoroborate) en présence d'acétonitrile, pour conduire aux composés de formule (I): 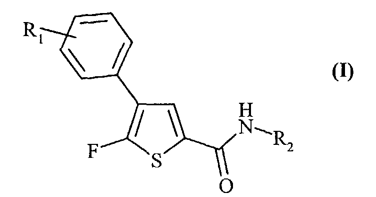 dans laquelle R₁ et R₂ sont tels que définis précédemment,
   composés de formule (I) qui peuvent éventuellement subir une hydrolyse basique dans le cas où R₂ représente un groupement U-R₄ dans lequel U représente une chaîne (C₁-C₄)alkylène linéaire substituée par un groupement choisi parmi (C₁-C₆)alkyloxycarbonyl- et (C₁-C₆)alkyloxycarbonylalkyl- pour conduire aux équivalents acide carboxylique de formule (I/a) cas particulier des composés de formule (I) : 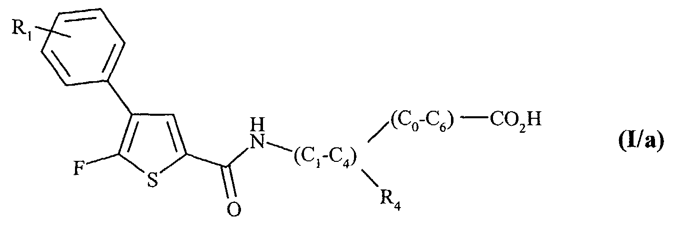 dans laquelle R₁ et R₄ sont tels que définis dans la formule (I),
   composés de formule (I/a) qui peuvent ensuite être traités dans une première étape par du chlorure d'oxalyle, puis dans une seconde étape avec une solution d'ammoniaque, ou une amine primaire ou secondaire, pour conduire aux composés de formule (I/b) cas particulier des composés de formule (I) : 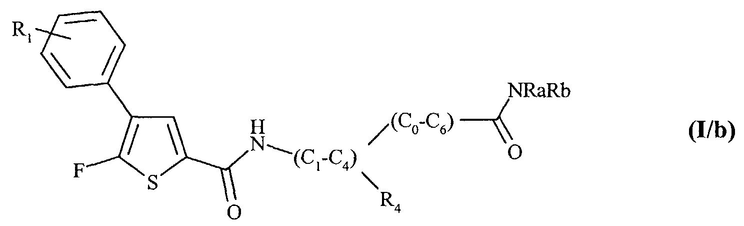 dans laquelle R₁ et R₄ sont tels que définis précédemment et, Ra et Rb représentent chacun un atome d'hydrogène ou un groupement (C₁-C₆)alkyle,
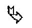 soit mis à réagir, après oxydation de la fonction aldéhyde suivie éventuellement d'une estérification de la fonction acide carboxylique obtenue, avec un acide phénylboronique correctement substitué tel que par un groupement nitro, pour conduire après deux étapes de synthèse classique de la substitution d'un groupement nitro par un atome d'halogène, aux composés de formule (VIII) : 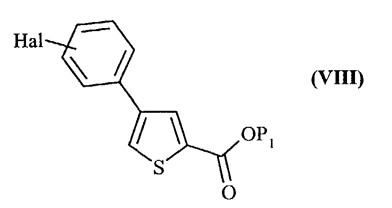 dans laquelle P₁ représente un atome d'hydrogène ou un groupement (C₁-C₆)alkyle linéaire ou ramifié, et Hal représente un atome d'halogène,
   composés de formule (VIII) qui sont alors traités par du 1-chlorométhyl-4-fluoro-1,4-diazoniabicyclo[2,2,2]octane bis(tetrafluoroborate) en présence d'acétonitrile, pour conduire aux composés de formule (IX) : 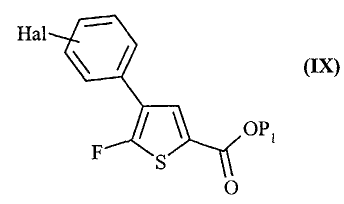 dans laquelle P₁ et Hal sont tels que définis précédemment,
   composés de formule (IX) qui sont alors mis à réagir en condition basique et en présence d'un catalyseur au palladium avec un composé de formule (X) : 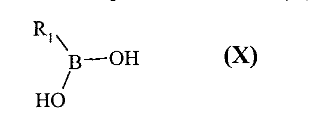 dans laquelle R₁ est tel que défini dans la formule (I),
   pour conduire aux composés de formule (XII) : 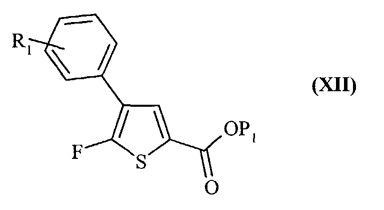 dans laquelle R₁ et P₁ sont tels que définis précédemment,
   composés de formule (XII) qui sont traités, dans des conditions de couplage peptidique en présence par exemple d'un agent de couplage et dans un milieu basique avec un composé de formule (VI) tel que défini précédemment pour conduire également aux composés de formule (I) : 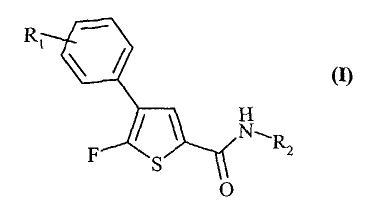 dans laquelle R₁ et R₂ sont tels que définis précédemment,
   composés de formule (I), qui sont purifiés, le cas échéant, selon une technique classique de purification, qui peuvent, si on le désire, être séparés en leurs différents isomères selon une technique classique de séparation, et que l'on transforme, le cas échéant, en leurs sels d'addition à un acide ou à une base pharmaceutiquement acceptable.

Selon une variante du procédé de l'invention la fluoration en position 5 du noyau thiènyle peut s'effectuer directement sur le thiènyle substitué en position 2 par une fonction carbaldéhyde.
Plus particulièrement les composés de formule (I) peuvent être obtenus à partir des composés de formule (II) : 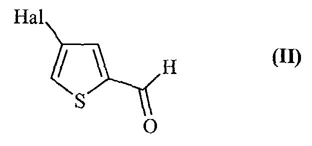 dans laquelle Hal représente un atome d'halogène,
composés de formule (II) qui sont mis à réagir, dans des conditions basiques de couplage au palladium, avec un composé de formule (III) : 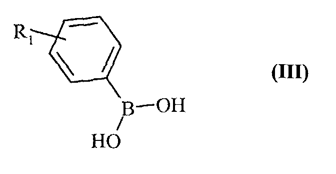 dans laquelle R₁ est tel que défini dans la formule (I),
pour conduire aux composés de formule (IVA) : 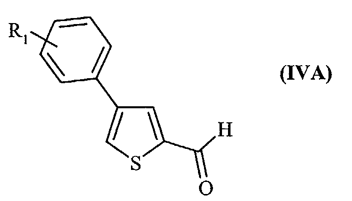 dans laquelle R₁ est tel que défini précédemment,
composés de formule (IVA) qui sont alors traités pendant 12 heures à 70°C par 1,95 équivalents de 1-chlorométhyl-4-fluoro-1,4-diazoniabicyclo[2,2,2]octane bis(tétrafluoroborate) en présence d'acétonitrile, pour conduire aux composés de formule (IVB) : 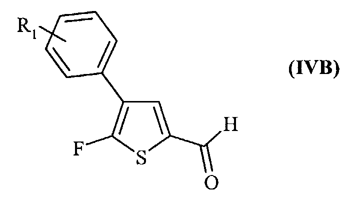 dans laquelle R₁ est tel que défini précédemment,
composés de formule (IVB) qui sont ensuite soumis à des conditions d'oxydation classiquement utilisées en synthèse organique pour conduire aux composés de formule (IVC) : 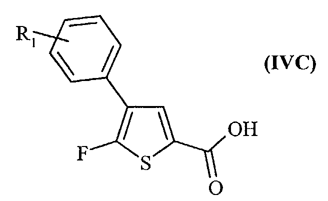 dans laquelle R₁ est tel que défini précédemment,
composés de formule (IVC) qui peuvent ensuite être traités de la même façon que les composés de formule (XII) telle que décrit dans le procédé précédent pour conduire également aux composés de formule (I).

Les composés de la présente invention, de par leurs propriétés pharmacologiques d'inhibiteurs de la MMP-12, sont utiles dans la prévention et le traitement des pathologies respiratoires telles que les broncho-pneumopathies chroniques obstructives (BPCO), l'emphysème, les bronchites chroniques, les inflammations pulmonaires chroniques, l'asthme, la mucoviscidose, la détresse respiratoire aiguë (ARDS), les allergies respiratoires dont la rhinite allergique, ainsi que les maladies liées à la production de TNFα incluant les maladies pulmonaires fibrotiques sévères, la sarcoïdose pulmonaire, et la silicose. Les composés de la présente invention montrent aussi, à un niveau moindre, une activité inhibitrice de la métalloprotéinase-13 (MMP-13) les rendant potentiellement utiles pour le traitement des pathologies impliquant cette enzyme, telles que le cancer, l'ostéoporose, l'ostéoarthrite, l'arthrite, l'arthrite rhumatoïde, l'athéorosclérose, la sclérose en plaques, l'insuffisance cardiaque.

D'une façon avantageuse les composés de la présente invention sont utiles pour la prévention et le traitement des broncho-pneumopathies chroniques obstructives, de l'emphysème et des bronchites chroniques.

Plus particulièrement les composés de la présente invention sont utiles pour le traitement de l'emphysème lié au tabagisme.

Selon une variante de l'invention, les composés de formule (I) sont utiles pour la prévention et le traitement de l'asthme.

La présente invention a également pour objet les compositions pharmaceutiques renfermant comme principe actif au moins un composé de formule (I), un de ses isomères ou un de ses sels d'addition à un acide ou à une base pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per- ou trans-cutanée, intravaginale, rectale, nasale, perlinguale ou respiratoire.

Les compositions pharmaceutiques selon l'invention pour les injections parentérales comprennent notamment les solutions stériles aqueuses et non aqueuses, les dispersions, les suspensions ou émulsions ainsi que les poudres stériles pour la reconstitution des solutions ou dispersions injectables.

Les compositions pharmaceutiques selon l'invention, pour les administrations orales solides, comprennent notamment les comprimés simples ou dragéifiés, les comprimés sublinguaux, les sachets, les gélules, les granules, et pour les administrations liquides orales, nasales ou buccales, comprennent notamment les émulsions, les solutions, les suspensions, les gouttes, les sirops et les aérosols.

Les compositions pharmaceutiques selon l'invention, pour les administrations par voie respiratoire, comprennent notamment des compositions sous forme de solutions pour aérosols ou de poudres pour inhalateurs. Lorsque les compositions sont des aérosols, pour l'usage d'aérosols liquides, les compositions peuvent être des solutions stériles stables ou des compositions solides dissoutes au moment de l'emploi dans de l'eau stérile apyrogène, dans du sérum physiologique ou tout autre véhicule pharmaceutiquement acceptable. Pour l'usage sous forme d'aérosols secs destinés à être directement inhalés, le principe actif est éventuellement finement divisé ou micronisé, et associé à un diluant ou véhicule inerte solide hydrosoluble.

Les compositions pharmaceutiques pour l'administration rectale sont préférentiellement des suppositoires, et celles pour l'administration per- ou trans-cutanée comprennent notamment les poudres, les aérosols, les crèmes, les pommades, les gels, et les patchs.

Les compositions pharmaceutiques citées précédemment illustrent l'invention mais ne la limitent en aucune façon.

Parmi les excipients ou véhicules inertes, non toxiques, pharmaceutiquement acceptables, on peut citer à titre indicatif et non limitatif les diluants, les solvants, les conservateurs, les agents mouillants, les émulsifiants, les agents dispersants, les liants, les agents gonflants, les agents désintégrants, les retardants, les lubrifiants, les absorbants, les agents de suspension, les colorants, les aromatisants, etc...

La posologie utile varie selon l'âge et le poids du patient, la voie d'administration, la composition pharmaceutique utilisée, la nature et la sévérité de l'affection, et la prise de traitements associés éventuels. La posologie s'échelonne de 1 mg à 1000 mg en une ou plusieurs prises par jour.

Les exemples suivants illustrent l'invention mais ne la limitent en aucune façon.

Les produits de départ utilisés sont des produits commerciaux ou des produits préparés selon des modes opératoires connus à partir de composés commerciaux ou connus par l'homme du métier. Les différentes préparations conduisent à des intermédiaires de synthèse utiles pour la préparation des composés de l'invention.

Les structures des composés décrits dans les exemples et dans les préparations ont été déterminées selon les techniques spectrophotométriques usuelles (infrarouge (IR), résonance magnétique nucléaire (RMN), spectrométrie de masse (SM) dont électrospray (ES), ...) et la pureté a été déterminée par chromatographie liquide à haute performance (HPLC).

Abréviations utilisées dans les modes opératoires :
- Sélectfluor™ : 1-Chlorométhyl-4-fluoro-1,4-diazoniabicyclo[2,2,2]octane bis(tétrafluoroborate) ;
- TOTU : O-[(éthoxycarbonyl)cyanométhylamino]-*N*-*N*-*N*'-*N*'-tetraméthyl uronium fluoroborate ;
- DME : 1,2-diméthoxyéthane (ou éthylène glycol diméthyl éther)
- TFA : acide trifluoroacétique
- HATU :O-(7-azabenzotriazol-1-yl)-*N*,*N*,*N*',*N*'-tétraméthyluronium hexafluorophosphate
- tBME : tert-butyl méthyl éther

### Préparation 1 : trans 4-(Aminométhyl)cyclohexanecarboxylate d'éthyle

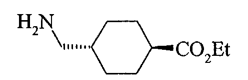

A une solution de 0,2 g d'acide *trans*-4-(aminométhyl)cyclohexane carboxylique dans 5 ml d'éthanol est ajouté 0,4 ml d'acide sulfurique. Le milieu réactionnel est porté à reflux pendant 17 heures, puis concentré sous pression réduite. Le résidu est repris par de l'acétate d'éthyle. La solution est basifiée jusqu'à pH 9 par addition d'une solution aqueuse de soude 1,0 M, lavée à l'eau, séchée sur sulfate de sodium, filtrée puis concentrée sous pression réduite permettant d'obtenir 0,129 g d'une huile jaune correspondant au produit attendu.
Rendement : 55%
SM : MH⁺ 186

### Exemple 1 : Acide 4-({[5-fluoro-4-(4-trifluorométhoxyphényl)-thièn-2-yl]carboxamido}méthyl)-cyclohexane carboxylique

### Stade 1 : 4-[4-(Trifluorométhoxy)phényl]thiophèn-2-carbaldéhyde

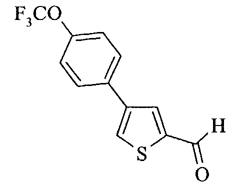

A une solution de 12,3 g de 4-bromothiophène-2-carbaldéhyde et 20,0 g d'acide 4-(trifluorométhoxy)phényl-boronique (1,2 équivalents) dans 70 ml de DME dégazé sont additionnés 84,9 ml (2,1 équivalents) d'une solution 2,0 M de phosphate de potassium et 2,8 g (0,03 équivalent) de tétrakis(triphénylphosphine)palladium(0). Le milieu réactionnel est agité 3 heures à 80°C puis concentré sous pression réduite. Le résidu obtenu est repris par de l'acétate d'éthyle. La solution est alors filtrée sur célite, lavée à l'eau, séchée sur sulfate de sodium, filtrée puis concentrée sous pression réduite. Une chromatographie sur gel de silice du résidu (cyclohexane/acétate d'éthyle : 9/1) permet d'isoler 15,05 g du produit attendu.
Rendement : 68%
RMN ¹H (CDCl₃) δ (ppm) : 10,0 (s, 1H), 8,0 (s, 1H), 7,80 (s, 1H), 7,55 (m,2H), 7,25 (m, 2H)

### Stade 2 : Acide 4-[4-(trifluorométhoxy)phéhyl]thiophèn-2-carboxylique

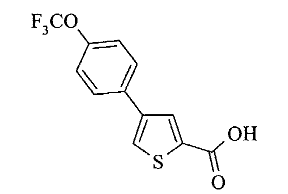

A une solution de 15,05 g du composé obtenu au stade 1 dans 200 ml d'éthanol sont additionnés 37,6 g (4 équivalents) de nitrate d'argent et 44,2 ml (8 équivalents) d'une solution aqueuse 1,0 M de soude. Le milieu réactionnel est agité 2 heures à 40°C, puis filtré sur célite et concentré sous pression réduite. La phase aqueuse est lavée par une solution d'acide chlorhydrique 1,0 M, extraite à l'acétate d'éthyle, séchée sur sulfate de sodium, filtrée et concentrée sous pression réduite, permettant d'obtenir 15,51 g d'une poudre beige correspondant au produit attendu.
Rendement : 97,4 %
SM : MH⁻ 287

### Stade 3 : Trans 4-({[4-(4-trifluorométhoxyphényl)-thièn-2-yl]carboxamido}méthyl)-cyclohexane carboxylate d'éthyle

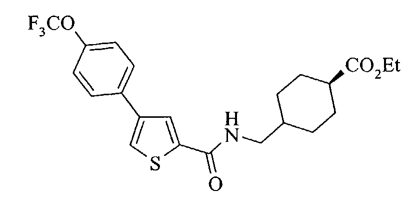

A une solution de 1,5 g du composé obtenu au stade 2 dans 15 ml de dichlorométhane anhydre sont ajoutés successivement 1,15 g du produit de la préparation 1 et 1,7 g de TOTU. Le milieu réactionnel est refroidi à 0°C puis 1,8 ml de *N*,*N*-diisopropyléthylamine sont ajoutés goutte à goutte. Après une nuit d'agitation à température ambiante, le milieu réactionnel est hydrolysé et extrait au dichlorométhane. Les phases organiques sont réunies, séchées sur sulfate de sodium, filtrées et évaporées sous pression réduite pour conduire après chromatographie sur gel de silice éluée par un gradient cyclohexane/acétate d'éthyle (95:5 à 80:20 de 5 en 5%) au produit attendu (2,124 g).
Rendement : 90 %
RMN ¹H (CDCl₃) δ (ppm) : 7,70 (s, 1H), 7,60 (m, 2H), 7,50 (s, 1H), 6,0 (bs, 1H), 4,10 (q, 2H), 3,40 (t, 2H), 2,25 (m, 1H), 2,0 (m, 2H), 1,90 (m, 2H), 1,60 (m, 1H), 1,40 (m, 2H), 1,25 (t, 3H), 1,10 (q, 2H)
HPLC : 95%

### Stade 4 : Trans 4-({(5-fluoro4-(4-trifluorométhoxyphényl)-thièn-2-yl]carboxamido}méthyl)-cyclohexane carboxylate d'éthyle

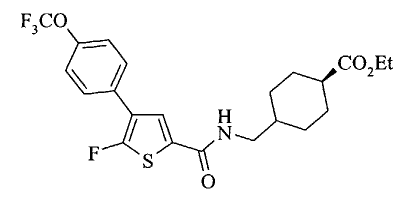

A une solution de 265 mg du composé obtenu au stade 3 dans 3,5 ml d'acétonitrile sont additionnés 207 mg (1 équivalent) de Sélectfluor™. Le milieu réactionnel est agité pendant 10 minutes à 70°C puis 3 heures à 50°C. Le brut réactionnel est hydrolysé, extrait au dichlorométhane, puis les phases organiques réunies sont lavées avec une solution saturée d'hydrogénocarbonate de sodium, séchées sur sulfate de magnésium, filtrées et enfin concentrées sous pression réduite, pour conduire à 218 mg d'un mélange correspondant au produit fluoré attendu contaminé par le produit de départ (proportion 1:1 déterminée par HPLC).

### Stade 5 : Acide trans 4-({[5-fluoro4-(4-trifluorométhoxyphényl)-thièn-2-yl]carboxamido}méthyl)-cyclohexane carboxylique

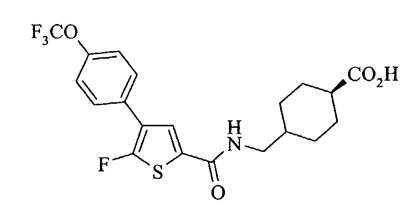

A une solution de 213 mg du mélange obtenu au stade 4 précédent dans 5 ml d'un mélange éthanol/eau (1/1) sont ajoutés 54 mg d'hydroxyde de lithium (5 équivalents). Le milieu réactionnel est agité pendant 4 heures à 50°C puis concentré sous pression réduite. Le solide obtenu est repris dans l'eau et acidifié avec une solution d'acide chlorhydrique 1,0 M jusqu'à pH 1. Le précipité formé est alors filtré, lavé successivement à l'eau et à l'éther puis séché pendant une nuit permettant ainsi d'obtenir 149 mg de mélange. Une chromatographie en phase inverse (conditions : colonne : C18, 21*50mm; mode : gradient A) H₂O + 0,1 % TFA et B) acétonitrile + 0,1% TFA de 30 % de B à 95 % en 13 min; débit : 30 ml/min; longueur d'onde : 214 nm) permet d'isoler 25 mg du produit attendu.
Rendement (stades 4 et 5) : 10 %
RMN ¹H (DMSO) δ (ppm) : 11,99 (bs, 1H), 8,54 (t, 1H), 7,90 (d,1H), 7,73 (d, 2H), 7,51 (d, 2H), 3,11 (t, 2H), 2,14 (t, 1H), 1,90 (d, 2H), 1,78 (d, 2H), 1,48 (m, 1H), 1,25 (m, 2H), 0,98 (q, 2H)
HPLC : 97,92 %
SM : MH⁺ 446

### Exemple 2 : Acide trans-4-({[5-fluoro-4-(4-[4-acétylphényl]-phényl)-thièn-2-yl]carboxanudo}méthyl)-cyclohexane carboxylique

### Stade 1 : 4-(4-Nitrophényl)thiophène-2-carboxylate de méthyle

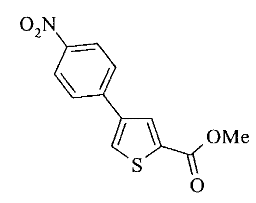

A une solution sous azote de 4-bromothiophène-2-carboxylate de méthyle dans 3,0 ml de DME dégazé sont ajoutés de l'acide (4-nitrophényl)boronique (1,2 équivalents), du tétrakis(triphénylphosphine)palladium(0) (0,03 équivalent) et une solution de phosphate de potassium à 2,0 M (2,1 équivalents). Le milieu réactionnel est ensuite agité pendant 3 heures à 80°C, dilué avec de l'acétate d'éthyle (20 ml), lavé à l'eau (2x15 ml), séché sur sulfate de sodium, filtré et concentré sous pression réduite. Une chromatographie sur gel de silice du résidu (dichlorométhane/méthanol : 98/2) permet d'isoler 1,94 g du produit attendu.
Rendement : 78 %
RMN ¹H (CDCl₃) δ (ppm) : 3,92 (s, 3H), 7,75 (d, 2H), 7,82 (s, 1H), 8,12 (s, 1H), 8,30 (d, 2H)

### Stade 2 : 4-(4-Aminophényl)thiophène-2-carboxylate de méthyle

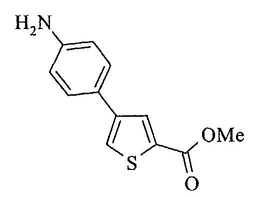

Une solution de 1,94 g du composé obtenu au stade 1 précédent dans 20 ml de méthanol contenant 194 mg de palladium sur charbon à 10% est agitée sous pression d'hydrogène (10 bars). Le milieu réactionnel est ensuite filtré sur célite et concentré sous pression réduite permettant d'obtenir 1,51 g du produit désiré.
Rendement : 88 %
RMN ¹H (DMSO) δ (ppm) : 3,82 (s, 3H), 5,22 (s, 2H), 6,60 (d, 2H), 7,42 (d, 2H), 7,90 (s, 1H), 8,05 (s, 1H)
SM : MH⁺ 234

### Stade 3 : 4-(4-Bromophéhyl)thiophène-2-carboxylate de méthyle

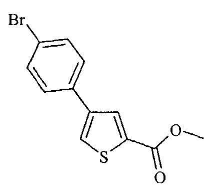

A une solution de 103 mg de produit obtenu lors du stade 2 précédent dans 1,5 ml d'eau est ajouté 0,6 ml d'acide bromhydrique concentré. Le milieu réactionnel est refroidi à 0°C, puis une solution de 35,5 mg de nitrite de sodium (1,1 équivalents) dans 0,5 ml d'eau est ajoutée goutte à goutte. Après 1 heure d'agitation à 0°C, une solution de 68 mg de bromure de cuivre dans 0,5 ml d'acide bromhydrique concentré est ajoutée goutte à goutte. Le milieu réactionnel est de nouveau agité pendant 1 heure à 0°C puis dilué avec de l'acétate d'éthyle (30 ml), lavé successivement avec de l'eau (3x15 ml), une solution saturée d'hydrogénocarbonate de sodium (15 ml), puis de l'eau (15 ml). La phase organique est séchée sur sulfate de sodium, filtrée puis concentrée sous pression réduite. Une chromatographie sur gel de silice du résidu (cyclohexane/acétate d'éthyle : 95/5) permet d'isoler 52 mg du produit désiré.
Rendement : 40 %
RMN ¹H (CDCl₃) δ (ppm) : 8,05 (s, 1H), 7,65 (s, 1H), 7,55 (d, 2H), 7,45 (d, 2H), 3,92 (s, 3H)
HPLC : 91,4 %

### Stade 4 : 4-(4-Bromophényl)-5-fluoro-thiophène-2-carboxylate de méthyle

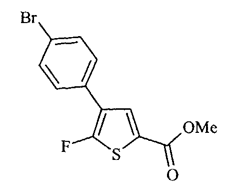

A une solution de 5 g du composé obtenu au stade 3 précédent dans 50 ml d'acétonitrile sont additionnés 5,95 g de Sélectfluor™ (1 équivalent). Le milieu réactionnel est agité 17 heures à 70°C. Après retour à température ambiante, le précipité formé est filtré et lavé à l'eau. Le solide ainsi obtenu correspond au dérivé fluoré contaminé par le produit de départ (proportion HPLC 60/40 en faveur de l'attendu). Le mélange est purifié par chromatographies successives sur gel de silice éluée par un mélange isochratique heptane/dichlorométhane (1:1) pour conduire au produit attendu (2,05 g).
Rendement : 40 %
RMN ¹H (DMSO) δ (ppm) : 8,02 (d, 1H), 7,67 (m, 4H), 3,85 (s, 3H)
HPLC : 100 %

### Stade 5 : 4-(4'-Acétyl-biphényl-4-yl)-5-fluoro-thiophène-2-carboxylate de méthyle

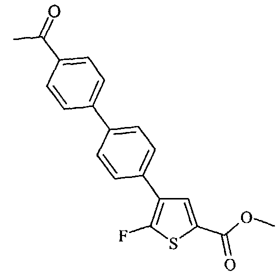

Le produit (310 mg) est obtenu selon le procédé du stade 1 de l'exemple 1, en utilisant comme co-substrat l'acide 4-acétylphénylboronique.
Rendement : 37 %
RMN ¹H (DMSO) δ (ppm) : 8,05 (m,3H), 7,87 (m, 6H), 3,87 (s, 3H), 2,65 (s, 3H)
HPLC : 98 %

### Stade 6 : Acide 4-(4'-acétyl-biphényl-4-yl)-5-fluoro-thiophène-2-carboxylique

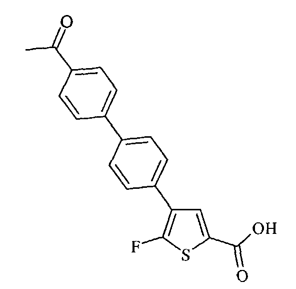

Le produit (230 mg) est obtenu selon le procédé du stade 5 de l'exemple 1, en utilisant comme substrat le produit obtenu au stade précédent.
Rendement : 77 %
RMN ¹H (DMSO) δ (ppm) : 8,05 (m, 3H), 7,85 (m, 6H), 6,0 (s, 3H)
HPLC : 80 %

### Stade 7 : trans-4-({[5-Fluoro-4-(4-[4-acétylphényl]-phényl)-thièn-2-yl]carboxamido}méthyl)-cyclohexane carboxylate d'éthyle

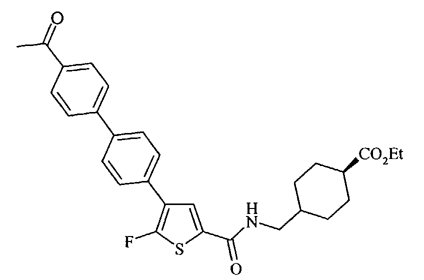

A une solution de 228 mg du composé précédent dans 15 ml de diméthylformamide anhydre sont ajoutés 255 mg de HATU (1 équivalent) puis 149 mg du produit de la préparation 1 (1,2 équivalents) et 0,2 ml de *N*-éthyl-*N*,*N*-diisopropylamine (2 équivalents). Le milieu réactionnel est agité pendant 3 heures à température ambiante puis hydrolysé. Le précipité qui se forme est filtré, lavé à l'eau puis séché. Une chromatographie sur gel de silice du solide obtenu (dichlorométhane/éthanol : 9/1) permet d'isoler le produit attendu (100 mg).
Rendement : 29 %
RMN ¹H (DMSO) δ (ppm) : 8,45 (t, 1H), 8,05 (m, 3H), 7,90 (m, 4H), 7,75 (d, 2H), 4,0 (m, 2H), 3,10 (t, 2H), 2,25 (t, 1H), 1,90 (d, 2H), 1,80 (d, 2H), 1,50 (m, 1H), 1,30 (q, 2H), 1,15 (m,3H), 1,0 (q, 2H)
HPLC : 76,0 %

### Stade 8 : Acide trans-4-({[5-fluoro-4-(4-[4-acétylphényl]-phényl)-thièn-2-yl]carboxamido}méthyl)-cyclohexane carboxylique

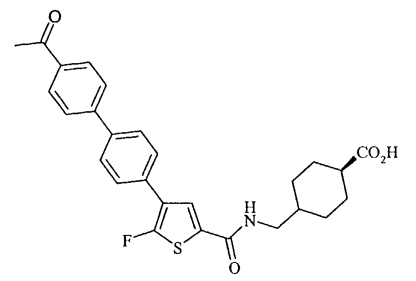

Le produit (21 mg) est obtenu selon le procédé du stade 5 de l'exemple 1, en utilisant comme substrat le produit obtenu au stade précédent.
Rendement : 47 %
RMN ¹H (DMSO) δ (ppm) : 12,0 (bs, 1H), 8,55 (t, 1H), 8,05 (d, 2H), 7,90 (d, 4H), 7,75 (d, 2H), 3,10 (t, 2H), 2,65 (s, 3H), 2,15 (t, 1H), 1,92 (d, 2H), 1,80 (d, 2H), 1,45 (m, 1H), 1,30 (q, 2H), 1,0 ( q, 2H)
HPLC : 86,6 %

### Exemple 3 : Acide 3-(R)-phényl-3-({5-fluoro-4-[4-(4-acétylphényl)-phényl]-thièn-2-yl}carboxamido)-propanoïque

### Stade 1 : 3-(R)-Phényl-3-({5-fluoro-4-[4-(4-acétylphényl)-phényl]-thièn-2-yl}carboxamido)-propanoate d'éthyle

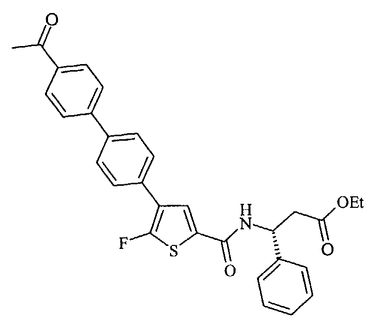

Le produit (70 mg) est obtenu selon le procédé du stade 7 de l'exemple 2, en utilisant comme co-substrat l'hydrochlorure de (R)-3-amino-3-phénylpropanoate d'éthyle.
Rendement (non optimisé) : 7%
HPLC : 98,0 %
SM : MH⁺ 516

### Stade 2 : Acide 3-(R)-phényl-3-({5-fluoro-4-[4-(4-acétylphényl)-phényl]-thièn-2-yl}carboxamido)-propanoïque

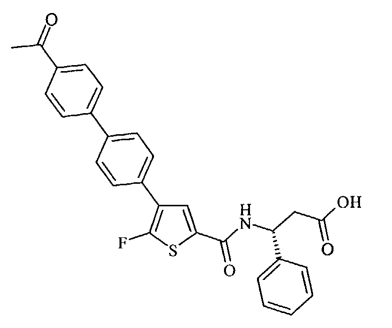

Le produit (25 mg) est obtenu selon le procédé du stade 5 de l'exemple 1, en utilisant comme substrat le produit obtenu au stade précédent.
Rendement : 38 %
RMN ¹H (DMSO) δ (ppm) : 12,41 (bs, 1H), 9,04 (bs, 1H), 8,14 (bs, 1H), 8,06 (d, 2H), 7,91 (t, 4H), 7,89 (d, 2H), 7,35 (m, 5H), 5,40 (dd, 1H), 2,84 (m, 2H), 2,62 (s, 3H)
HPLC : 98,32 %

### Example 4 : Acide 3-(R)-phényl-3-({5-fluoro-4-[4-trifluorométhoxyphényl]-thièn-2-yl}carboxamido)-propanoïque

### Stade 1 : 5-Fluoro-4-[4-(trifluorométhoxy)phényl]thiophèn-2-carbaldéhyde

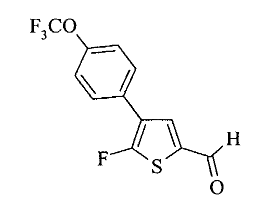

A une solution de 5 g de 4-[4-(trifluorométhoxy)phényl]thiophène-2-carboxaldéhyde obtenu au stade 1 de l'exemple 1 dans 140 ml d'acétonitrile sont additionnés sous azote 12,7 g (1,95 équivalents) de Sélectfluor™. L'ensemble réactionnel est chauffé à 70 °C pendant 16 heures, puis refroidi et concentré sous pression réduite. Le résidu repris dans 50 ml de tBME est hydrolysé et extrait plusieurs fois au tBME. Les phases organiques réunies sont séchées sur sulfate de magnésium, filtrées, évaporées sous vide pour conduire à une huile brune qui est purifiée sur gel de silice (heptane/tBME : 98/2). Les fractions désirées sont réunies pour conduire à 1,8 g de produit sous forme de poudre blanche.
Rendement : 30 %
RMN ¹H (DMSO) δ (ppm) : 9,88 (s, 1H), 8,35 (s, 1H), 7,82 (d, 2H), 7,50 (d, 2H)

### Stade 2 : Acide 5-fluoro-4-[4-(Trifluorométhoxy)phényl]thiophèn-2-yl-carboxylique

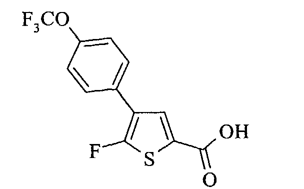

Le produit (900 mg) est obtenu selon le procédé du stade 2 de l'exemple 1, en utilisant comme substrat le produit obtenu au stade précédent.
Rendement : 47 %
RMN ¹H (DMSO) δ (ppm) : 13,50 (bs, 1H), 7,93 (s, 1H), 7,82 (d, 2H), 7,50 (d, 2H)

### Stade 3 : 3-(R)-Phényl-3-({5-fluoro-4-[4-(trifluorométhoxy)-phéhyl]-thièn-2-yl}carboxamido)-propanoate d'éthyle

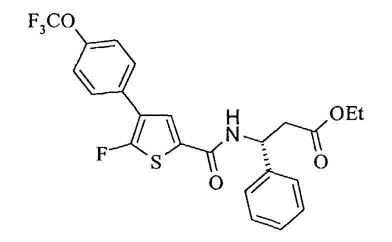

Le produit (1,054 g) est obtenu selon le procédé du stade 7 de l'exemple 2, en utilisant comme co-substrat l'hydrochlorure de (R)-3-amino-3-phénylpropanoate d'éthyle.
Rendement : 84 %
HPLC : 99,0 %
SM : MH⁺ 482, MH- 480

### Stade 4 : Acide 3-(R)-phényl-3-({5-fluoro-4-[4-(trifluorométhoxy)phényl]-thièn-2-yl}carboxamido)-propanoïque

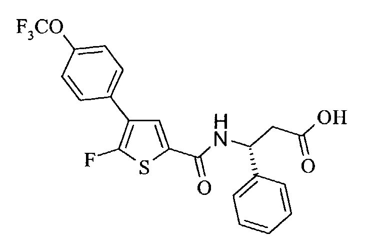

Le produit (771 mg) est obtenu selon le procédé du stade 5 de l'exemple 1, en utilisant comme substrat le produit obtenu au stade 3 précédent.
Rendement : 77 %
RMN ¹H (DMSO) δ (ppm) : 12,34 (bs, 1H), 8,98 (d, 1H), 8,06 (s, 1H), 7,74 (d, 2H), 7,51 (d, 2H), 7,36 (m, 4H), 7,26 (m, 1H), 5,38 (m, 1H), 2,83 (m, 2H)
HPLC : 98,68 %
SM : MH⁻ 452

### Example 5 : 3-(R)-Phényl-3-({5-fluoro-4-[4-(4-acétylphényl)-phényl]-thièn-2-yl}carboxamido)-propanamide,

### Stade 1 : Chlorure de 3-(R)-phényl-3-({5-fluoro-4-[4-trifluorométhoxyphényl]-thièn-2-yl} carboxamido)-propanoïque

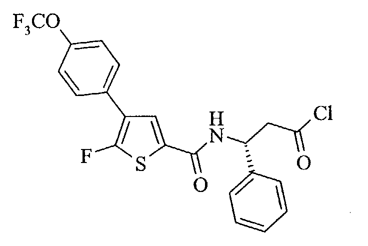

A une solution de 681 mg du composé obtenu au stade 4 de l'exemple 4 dans 10 ml de dichlorométhane anhydre additionnée de quelques gouttes de diméthylformamide sont ajoutés goutte à goutte, à température ambiante, 2,54 ml (4 équivalents) de chlorure d'oxalyle. Après 45 minutes de réaction, le milieu réactionnel est concentré sous pression réduite permettant d'obtenir une huile jaune correspondant au produit attendu utilisé sous forme brute au cours du stade suivant.

### Stade 2 : 3-(R)-Phényl-3-({5-fluoro-4-[4-trifluorométhoxyphényl]-thièn-2-yl}carboxamido)-propanamide

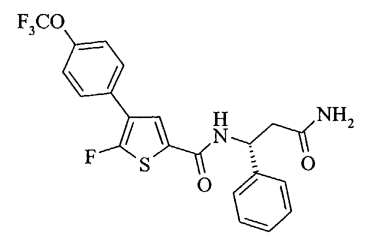

A la solution obtenue au stade 1 précédent reprise dans 10 ml de tétrahydrofurane anhydre est additionné à 0 °C un large excès d'une solution aqueuse d'ammoniaque à 28%. Le bain glacé est retiré et l'ensemble est agité pendant une nuit à température ambiante. Le brut réactionnel est concentré sous pression réduite, hydrolysé et extrait au dichlorométhane. Les phases organiques sont réunies, séchées sur sulfate de sodium, filtrées et concentrées sous pression réduite pour conduire à un résidu qui est purifié par chromatographie sur gel de silice (cyclohexane/ acétate d'éthyle : 80:20). Le produit désiré (146 mg) est obtenu sous forme d'une poudre blanche.
Rendement: 21,5 %
RMN ¹H (DMSO) δ (ppm) : 8,93 (d, 1H), 8,06 (d, 1H), 7,74 (d, 2H), 7,52 (d, 2H), 7,35 (m, 5H), 7,24 (t, 1H), 6,85 (s, 1H), 5,41 (q, 1H), 2,66 (m, 2H)
HPLC : 99,8 %
SM : MH⁺ 453, MH- 451

### Exemple 6 : Acide (R) 3-phényl-3-({5-fluoro-4-[4-(4-pyridyl)-phényl]-thièn-2-yl}carboxamido)-propanoïque

### Stade 1 : [4-(4-Pyridyl)-phényl]-5-fluoro-thiophène-2-carboxylate de méthyle

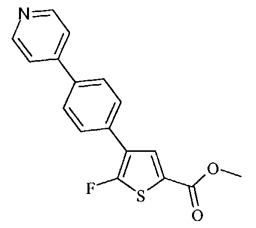

Le produit (260 mg) est obtenu selon le procédé du stade 1 de l'exemple 1, en utilisant comme co-substrat l'acide 4-pyridylboronique.
Rendement : 34 %
HPLC : 98,69 %
MS : MH⁺ 314

### Stade 2 : Chlorhydrate d'acide [4-(4-pyridyl)phenyl]-5-fluoro-thiophène-2-carboxylique

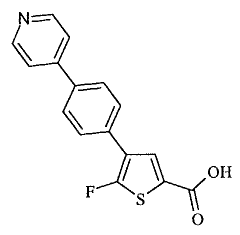

Le produit (223 mg) est obtenu sous forme de sel selon le procédé du stade 5 de l'exemple 1, en utilisant comme substrat le produit obtenu au stade précédent.
Rendement : 80 %
HPLC : 98,28 %
MS : MH⁺ 300, MH- 298

### Stade 3 : 3-(R)-Phényl-3-({5-fluoro-4-[4-(4-pyridyl)-phényl]-thièn-2-yl}carboxamido)-propanoate d'éthyle

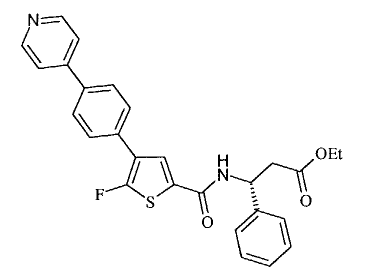

Le produit (220 mg) est obtenu selon le procédé du stade 7 de l'exemple 2, en utilisant comme co-substrat l'hydrochlorure de (R)-3-amino-3-phénylpropanoate d'éthyle.
Rendement : 70 %
HPLC : 95,07 %
MS : MH⁺ 475, MH⁻ 473

### Stade 4 : Chlorhydrate d'acide 3-(R)-phényl-3-({5-fluoro-4-[4-(4-pyridyl)-phényl]-thièn-2-yl}carboxamido)-propanoïque

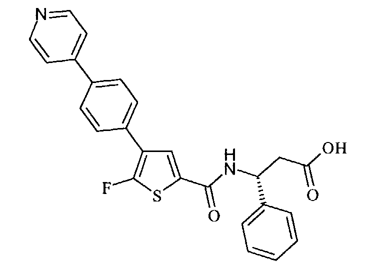

Le produit (166 mg) est obtenu sous forme de sel selon le procédé du stade 5 de l'exemple 1, en utilisant comme substrat le produit obtenu au stade précédent.
Rendement : 75 %
RMN ¹H (DMSO) δ (ppm) : 12,25 (bs, 1H), 9,18 (d, 1H), 8,94 (d, 2H), 8,37 (d, 2H), 8,31 (s, 1H), 8,17 (d, 2H), 7,87 (d, 2H), 7,44 (d, 2H), 7,35 (t, 2H), 7,26 (t, 1H), 5,40 (q, 1H), 2,88 (m, 2H)
HPLC : 98,56 %
SM : MH⁺ 447

### Exemple 7 : Etudes pharmacologiques des composés de l'invention:

### Evaluation in vitro de l'activité inhibitrice des composés de l'invention sur la MMP-12 :

L'activité inhibitrice des composés de formule (I) sur la métalloprotéinase-12 est évaluée en testant la capacité des composés de l'invention à inhiber la protéolyse d'un peptide substrat de la MMP-12.
Le peptide substrat utilisé (peptide-1 fluorigénique : FP-1) dans le test présente la séquence suivante : Mca-Pro-Leu-Gly-Leu-Dap(Dnp)-Ala-Arg-NH₂
L'activité inhibitrice d'un composé de formule (I) est exprimée en valeur d'IC₅₀, qui représente la concentration en inhibiteur pour laquelle un taux d'inhibition de 50% de la métalloprotéinase est observé.

La réaction commence par l'addition séquentielle de 41 µl de substrat FP-1 (concentration finale de 10 µM) à une solution tampon de 50 mM de Tris-HCl et 10 mM de CaCl₂, et contenant 5 mM d'acide hydroxamique et 5 µl de l'enzyme diluée dans une solution tampon 0,005% Brij-35. Les microplaques sont incubées pendant 20 minutes à température ambiante. Les composés de l'invention sont testés à des concentrations variants de 0,3 à 30 µM.

La mesure de la quantité de protéolyse du substrat peptidique est suivie par une mesure d'absorbance à 405 nm en utilisant un spectrophotomètre de microplaques, à la température ambiante. Les valeurs d'IC₅₀ sont calculées à partir de courbes dans lesquelles le pourcentage de l'activité catalytique relative au contrôle est représenté sur l'axe des X et la concentration en inhibiteur est représentée sur l'axe des Y.

Le test décrit ci-dessus pour l'inhibition de la MMP-12 est adapté et utilisé pour déterminer la capacité des composés de formule (I) à inhiber les métalloprotéinases MMP-1, MMP-2, MMP-3, MMP-7, MMP-9, MMP-13 et MMP-14. Les résultats obtenus montrent que les composés de l'invention ont généralement des valeurs d'IC₅₀ pour la MMP-12 qui sont de 5 à plus de 100 fois plus faibles que les valeurs d'IC₅₀ obtenues pour le même composé avec les autres métalloprotéinases testées, prouvant ainsi leur capacité d'inhibition sélective vis-à-vis de la métalloprotéinase-12 (MMP-12). Plus spécifiquement les composés de la présente invention montrent généralement une sélectivité avec un facteur supérieur à 50 vis-à-vis des métalloprotéinases mentionnées précédemment, excepté à l'égard de la MMP-13. Ainsi, les composés de la présente invention montrent également une activité inhibitrice sur la MMP-13 permettant également d'utiliser les compositions pharmaceutiques contenenant un ou plusieurs composés de l'invention pour le traitement des pathologies liées à une activité de la MMP-13. Parmi ces pathologies, on peut citer à titre indicatif et non limitatif, le cancer, l'ostéoporose, l'ostéoarthrite, l'arthrite, l'arthrite rhumatoïde, l'athéorosclérose, la sclérose en plaques, l'insuffisance cardiaque, l'asthme et les broncho-pneumopathies chroniques obstructives.

A titre d'exemple et sans limitation de l'invention, le tableau présente quelques résultats d'activité des composés de l'invention vis-à-vis de la MMP-12 et de la MMP-13.

| Exemple | IC₅₀ (µM) MMP-12 | IC₅₀ (µM) MMP-13 |
|---|---|---|
| 1 | 0,140 | 2,8 |
| 2 | 0,007 | 0,038 |
| 3 | 0,024 | 0,085 |

## Revendications

1. Composés de formule (I) : 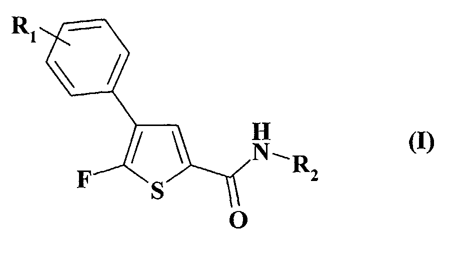 dans laquelle :
• R₁ représente un groupement choisi parmi halogéno(C₁-C₆)alkoxy, (C₁-C₆)alkyle, (C₁-C₆)alkoxy, (C₁-C₆)alkylthio, et -R₃ dans lequel :
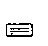 R₃ représente un groupement choisi parmi phényle, cyclohexyle, et un hétérocycle, chacun de ces groupements étant éventuellement substitués par un à deux groupements, identiques ou différents, indépendamment l'un de l'autre choisis parmi halogène, halogéno(C₁-C₆)alkyl-, halogéno(C₁-C₆)alkoxy-, (C₁-C₆)alkyle, cyano, cyano(C₁-C₆)alkyl-, hydroxy, (C₁-C₆)alkoxy, phénoxy, (C₁-C₆)alkyl-SO₂-, (C₁-C₆)alkylcarbonyl, benzoyl, hydroxy(C₁-C₆)alkyl-, et amino éventuellement substitué par un ou deux groupements (C₁-C₆)alkyle identiques ou différents ;
• R₂ représente un groupement -U-R₄ dans lequel :
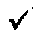 U représente une chaîne (C₁-C₄)alkylène linéaire éventuellement substituée par un groupement choisi parmi carboxy, carboxy(C₁-C₆)alkyl-, (C₁-C₆)alkyloxycarbonyl, (C₁-C₆)alkyloxycarbonyl(C₁-C₆)alkyl-, aminocarbonyl et aminocarbonyl(C₁-C₆)alkyl dans lesquels la partie amino est éventuellement substituée par un ou deux groupements (C₁-C₆)alkyl identiques ou différents,
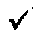 R₄ représente un groupement phényle, cyclohexyle, ou morpholin-4-yl chacun de ces groupements étant éventuellement substitué par un ou deux groupements, identiques ou différents, indépendamment l'un de l'autre choisis parmi halogène, -OR₅, -CO₂R₅, -W-CO₂R₅, dans lesquels :
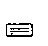 R₅ représente un atome d'hydrogène ou un groupement (C₁-C₆)alkyle,
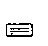 W représente une chaîne (C₁-C₆)alkylène linéaire ou ramifiée,
leurs isomères, ainsi que leurs sels d'addition à un acide ou à une base pharmaceutiquement acceptable,

2. Composés de formule (I) selon la revendication 1 **caractérisés en ce que** le groupement phényle des composés de formule (I) est substitué par un groupement R₁ tel que défini dans la formule (I) situé en position *para,* leurs isomères, ainsi que leurs sels d'addition à un acide ou à une base pharmaceutiquement acceptable.

3. Composés de formule (I) selon l'une quelconque des revendications 1 ou 2 **caractérisés en ce que** R₁ représente un groupement choisi parmi trifluorométhoxy, 4-acétylphényle, 4-pyridyle, 3-pyridyle, N-pyrrolydinyle, 1-méthylpyrrol-3-yl, 3,6-dihydro-2H-pyridin-1-yl, 2-hydroxyphényle, et la 2-hydroxy-4-pyridyle leurs isomères, ainsi que leurs sels d'addition à un acide ou à une base pharmaceutiquement acceptable.

4. Composés de formule (I) selon l'une quelconque des revendications 1 à 3 **caractérisés en ce que** R₁ représente un groupement choisi parmi trifluorométhoxy, 4-acétylphényle et 4-pyridyle, leurs isomères, ainsi que leurs sels d'addition à un acide ou à une base pharmaceutiquement acceptable.

5. Composés de formule (I) selon la revendication 1 **caractérisés en ce que** R₂ représente un groupement -U-R₄ dans lequel U représente une chaîne (C₁-C₂)alkylène linéaire et R₄ représente un groupement cyclohexyle substitué en position 4- par un groupement carboxy, leurs isomères, ainsi que leurs sels d'addition à un acide ou à une base pharmaceutiquement acceptable.

6. Composés de formule (I) selon la revendication 5 **caractérisés en ce que** le groupement carboxy situé en position 4 du groupement cyclohexyle est de stéréochimie trans, leurs isomères, ainsi que leurs sels d'addition à un acide ou à une base pharmaceutiquement acceptable.

7. Composés de formule (I) selon la revendication 1 **caractérisés en ce que** R₂ représente un groupement -U-R₄ dans lequel U représente un groupement méthylène substitué par un groupement carboxyméthyle ou aminocarbonylméthyle, et R₄ représente un groupement phényle, leurs isomères, ainsi que leurs sels d'addition à un acide ou à une base pharmaceutiquement acceptable.

8. Composés de formule (I) selon la revendication 6 **caractérisés en ce que** l'atome de carbone portant le groupement R₄ est de configuration absolue (R), ainsi que leurs sels d'addition à un acide ou à une base pharmaceutiquement acceptable.

9. Composés de formule (I) selon la revendication 1 qui sont 1' :
- acide *trans* 4-({[5-fluoro-4-(4-trifluorométhoxyphényl)-thièn-2-yl]carboxamido}méthyl)-cyclohexane carboxylique
- acide trans-4-({[5-fluoro-4-(4-[4-acétylphényl]-phényl)-thièn-2-yl]carboxamido}méthyl)-cyclohexane carboxylique
- acide 3-(*R*)-phényl-3-({5-fluoro-4-[4-(4-acétylphényl)-phényl]-thièn-2-yl}carboxamido)-propanoïque,
- acide 3-(*R*)-phényl-3-({5-fluoro-4-[4-trifluorométhoxyphényl]-thièn-2-yl}carboxamido)-propanoïque,
- 3-(*R*)-phényl-3-({5-fluoro-4-[4-(4-acétylphényl)-phényl]-thièn-2-yl}carboxamido)-propanamide,
- et le chlorhydrate d'acide 3-(R)-phényl-3-({5-fluoro-4-[4-(4-pyridyl)-phényl]-thièn-2-yl} carboxamido)-propanoïque,
leurs isomères ainsi que leurs sels d'addition à un acide ou à une base pharmaceutiquement acceptable.

10. Procédé de préparation des composés de formule (I) **caractérisé en ce qu'**on utilise comme produit de départ un composé de formule (II) : 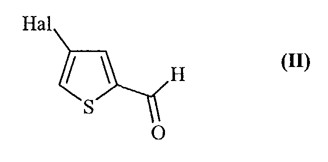 dans laquelle Hal représente un atome d'halogène,
composés de formule (II) qui sont :
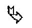 soit mis à réagir, dans des conditions basiques de couplage au palladium, avec un composé de formule (III) : 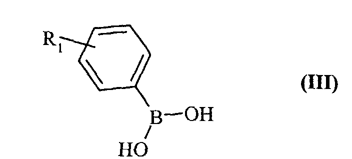 dans laquelle R₁ est tel que défini dans la formule (I), pour conduire aux composés de formule (IV) : 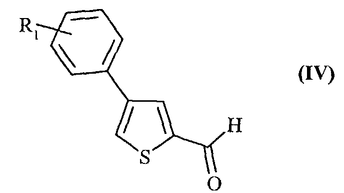 dans laquelle R₁ est tel que défini précédemment,
composés de formule (IV) qui sont soumis à des conditions d'oxydation en présence, par exemple, de nitrate d'argent en milieu basique et polaire, pour conduire aux composés de formule (V) : 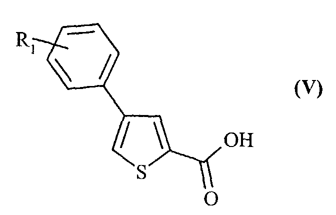 dans laquelle R₁ est tel que défini précédemment,
composés de formule (V) qui sont traités, dans des conditions de couplage peptidique en présence par exemple d'un agent de couplage et dans un milieu basique avec un composé de formule (VI) :
H₂N-R₂ (VI)
dans laquelle R₂ est tel que défini dans la formule (I) :
pour conduire aux composés de formule (VII) : 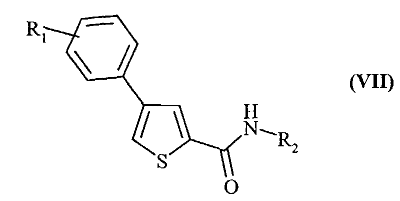 dans laquelle R₁ et R₂ sont tels que définis précédemment,
composés de formule (VII) qui sont alors traités par du 1-chlorométhyl-4-fluoro-1,4-diazoniabicyclo[2,2,2]octane bis(tétrafluoroborate) en présence d'acétonitrile, pour conduire aux composés de formule (I): 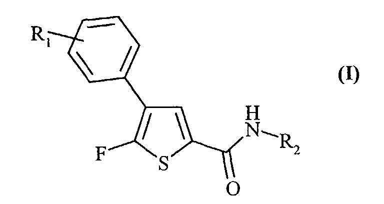 dans laquelle R₁ et R₂ sont tels que définis précédemment,
composés de formule (I) qui peuvent éventuellement subir une hydrolyse basique dans le cas où R₂ représente un groupement U-R₄ dans lequel U représente une chaîne (C₁-C₄)alkylène linéaire substituée par un groupement choisi parmi (C₁-C₆)alkyloxycarbonyl- et (C₁-C₆)alkyloxycarbonylalkyl- pour conduire aux équivalents acide carboxylique de formule (I/a) cas particulier des composés de formule (I) : 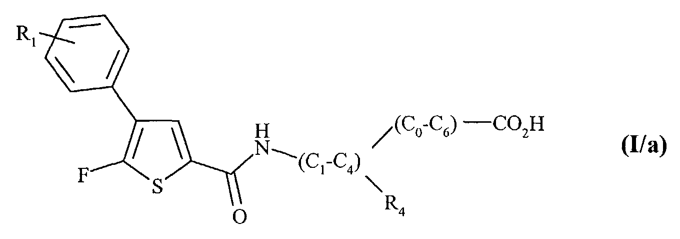 dans laquelle R₁ et R₄ sont tels que définis dans la formule (I),
composés de formule (I/a) qui peuvent ensuite être traités dans une première étape par du chlorure d'oxalyle, puis dans une seconde étape avec une solution d'ammoniaque, ou une amine primaire ou secondaire, pour conduire aux composés de formule (I/b) cas particulier des composés de formule (I) : 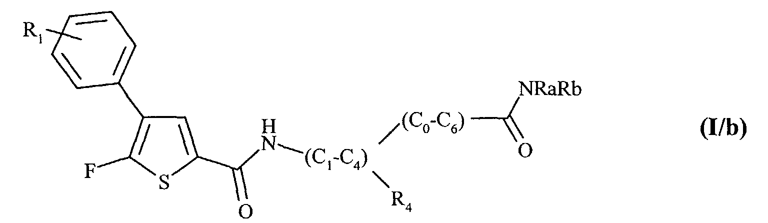 dans laquelle R₁ et R₄ sont tels que définis précédemment et, Ra et Rb représentent chacun un atome d'hydrogène ou un groupement (C₁-C₆)alkyle,
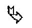 soit mis à réagir, après oxydation de la fonction aldéhyde suivie éventuellement d'une estérification de la fonction acide carboxylique obtenue, avec un acide phénylboronique correctement substitué tel que par un groupement nitro, pour conduire après deux étapes de synthèse classique de la substitution d'un groupement nitro par un atome d'halogène, aux composés de formule (VIII) : 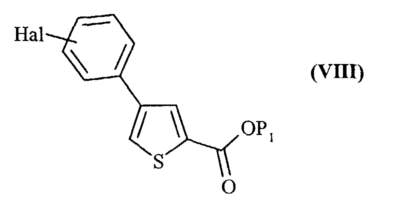 dans laquelle P₁ représente un atome d'hydrogène ou un groupement (C₁-C₆)alkyle linéaire ou ramifié, et Hal représente un atome d'halogène,
composés de formule (VIII) qui sont alors traités par du 1-chlorométhyl-4-fluoro-1,4-diazoniabicyclo[2,2,2]octane bis(tétrafluoroborate) en présence d'acétonitrile, pour conduire aux composés de formule (IX) : 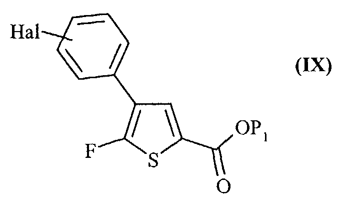 dans laquelle P₁ et Hal sont tels que définis précédemment,
composés de formule (IX) qui sont alors mis à réagir en condition basique et en présence d'un catalyseur au palladium avec un composé de formule (X) : 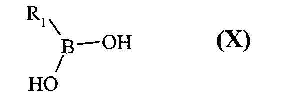 dans laquelle R₁ est tel que défini dans la formule (I), pour conduire aux composés de formule (XII) : 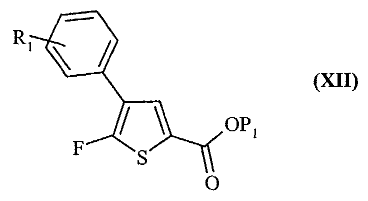 dans laquelle R₁ et P₁ sont tels que définis précédemment,
composés de formule (XII) qui sont traités, dans des conditions de couplage peptidique en présence par exemple d'un agent de couplage et dans un milieu basique avec un composé de formule (VI) tel que défini précédemment pour conduire également aux composés de formule (I) : 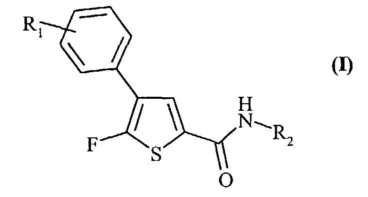 dans laquelle R₁ et R₂ sont tels que définis précédemment,
composés de formule (I), qui sont purifiés, le cas échéant, selon une technique classique de purification, qui peuvent, si on le désire, être séparés en leurs différents isomères selon une technique classique de séparation, et que l'on transforme, le cas échéant, en leurs sels d'addition à un acide ou à une base pharmaceutiquement acceptable.

11. Procédé de préparation des composés de formule (I) **caractérisé en ce qu'**on utilise comme produit de départ un composé de formule (II) : 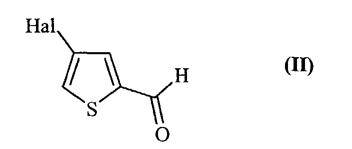 dans laquelle Hal représente un atome d'halogène,
composés de formule (II) qui sont mis à réagir, dans des conditions basiques de couplage au palladium, avec un composé de formule (III) : 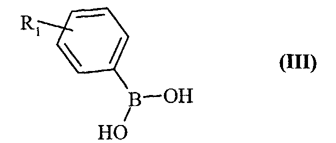 dans laquelle R₁ est tel que défini dans la formule (I),
Pour conduire aux composés de formule (IVA) : 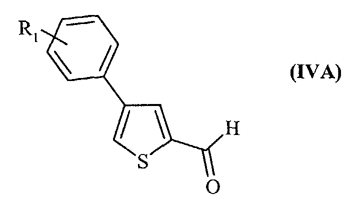 dans laquelle R₁ est tel que défini précédemment,
composés de formule (IVA) qui sont alors traités pendant 12 heures à 70°C par 1,95 équivalents de 1-chlorométhyl-4-fluoro-1,4-diazoniabicyclo[2,2,2]octane bis(tétrafluoroborate) en présence d'acétonitrile, pour conduire aux composés de formule (IVB) : 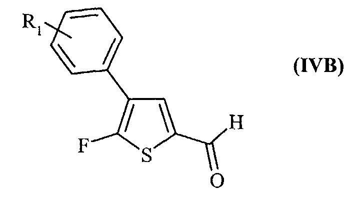 dans laquelle R₁ est tel que défini précédemment,
composés de formule (IVB) qui sont ensuite soumis à des conditions d'oxydation classiquement utilisées en synthèse organique pour conduire aux composés de formule (IVC) : 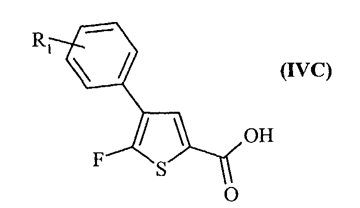 dans laquelle R₁ est tel que défini précédemment,
composés de formule (IVC) qui peuvent ensuite être traités de la même façon que les composés de formule (XII) telle que décrit dans le procédé précédent pour conduire également aux composés de formule (I).

12. Compositions pharmaceutiques contenant comme principe actif au moins un composé de formule (I) selon l'une quelconque des revendications 1 à 9, seul ou en combinaison avec un ou plusieurs excipients ou véhicules inertes, non toxiques, pharmaceutiquement acceptables

13. Compositions pharmaceutiques selon la revendication 12 utiles pour la prévention ou le traitement des pathologies nécessitant l'action d'un inhibiteur de métalloprotéinase-12 et/ou de métalloprotéinase-13.

14. Compositions pharmaceutiques selon la revendication 12 utiles pour la prévention ou le traitement des pathologies nécessitant l'action d'un inhibiteur de métalloprotéinase-12

15. Compositions pharmaceutiques selon la revendication 12 utiles pour la prévention ou le traitement des pathologies respiratoires liées à une inhibition de la métalloprotéinase-12, choisies parmi les broncho-pneumopathies chroniques obstructives (BPCO), l'emphysème, les bronchites chroniques, les inflammations pulmonaires chroniques, l'asthme, la mucoviscidose, la détresse respiratoire aiguë (ARDS), les allergies respiratoires dont la rhinite allergique, ainsi que les maladies liées à la production de TNFα incluant les maladies pulmonaires fibrotiques sévères, la sarcoïdose pulmonaire, et la silicose.

16. Compositions pharmaceutiques selon la revendication 12 utiles pour la prévention ou le traitement des pathologies liées à une inhibition de la métalloprotéinase-13 choisies parmi le cancer, l'ostéoporose, l'ostéoarthrite, l'arthrite, l'arthrite rhumatoïde, l'athéorosclérose, la sclérose en plaques, l'insuffisance cardiaque.

17. Compositions pharmaceutiques selon la revendication 12 utiles pour la prévention ou le traitement des broncho-pneumopathies chroniques obstructives, de l'emphysème et des bronchites chroniques.

18. Compositions pharmaceutiques selon la revendication 12 utiles pour la prévention ou le traitement de l'emphysème lié au tabagisme.

19. Compositions pharmaceutiques selon la revendication 12 utiles pour la prévention ou le traitement de l'asthme.
